(19) Europäisches Patentamt
European Patent Office
Office européen des brevets

(11) Publication number: **0 489 406 A1**

(12) **EUROPEAN PATENT APPLICATION**

(21) Application number: **91120774.4**

(51) Int. Cl.$^5$: **C07C 25/18**, C07C 17/12

(22) Date of filing: **03.12.91**

(30) Priority: **03.12.90 US 620844**

(43) Date of publication of application:
**10.06.92 Bulletin 92/24**

(84) Designated Contracting States:
**BE DE ES FR GB IT NL**

(71) Applicant: **ETHYL CORPORATION**
**451 Florida Boulevard**
**Baton Rouge, Louisiana 70801(US)**

(72) Inventor: **Hussain, Saadat**
**5321 Highland Ridge Drive**
**Baton Rouge, Louisiana 70817(US)**

(74) Representative: **Schwabe - Sandmair - Marx**
**Stuntzstrasse 16**
**W-8000 München 80(DE)**

(54) **Decabromodiphenylalkane process.**

(57) This invention relates to a process for preparing a white or at least near white product which is predominant in decabromodiphenylalkane and which has an average bromine number of at least about 9.0.

EP 0 489 406 A1

This invention relates to a process for preparing a product predominant in decabromodiphenylalkane.

Polybromodiphenylalkanes, e.g., decabromodiphenylethane and decabromodiphenylmethane,are known flame retardants for use in polyolefin and in polystyrenic-based formulations. On a commercial basis, the polybromodiphenylalkane would be supplied to the formulation as a product predominant in the polybromodiphenyl-alkane selected. The product would have a form and an impurity content which would be characteristic of the process used to produce it. If the product's physical characteristics, e.g., thermal stability, limit the formulation's processability, then the processor's desire for the product is limited at best. If the product's color is not white or at least near white, the product will be suitable for use in some formulations, however, the product's use may not be acceptable in formulations calling for a white or light color.

The process of this invention yields a white or near white product which is predominant in decabromodiphenylalkane and has an average bromine number of at least about 9.0. The process comprises: forming a stirrable reaction mass by feeding bromine to a reaction vessel containing diphenylalkane, which diphenyl-alkane is, at least initially, in solid form, and a bromination catalyst, the bromine (i) containing about 10 ppm or less impurities, and (ii) being charged in an amount which provides from 18 to 30 moles of bromine per mole of diphenylalkane; maintaining the reaction mass at a temperature which is less than or equal to about 15 °C during the feeding; subsequent to the feeding, obtaining a reaction mass temperature within the range of from 50 °C to 60 °C; and recovering from the reaction mass the decabromodiphenylalkane predominant product.

The diphenylalkane portion of the feed solution can be represented by the formula:

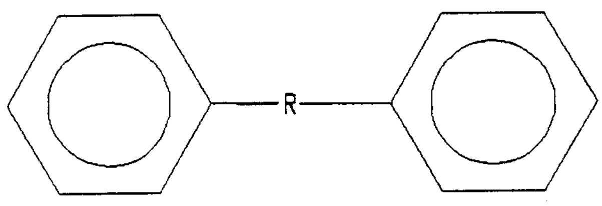

wherein R is alkylene group containing 1 to 10 carbon atoms. Preferred R groups are methylene and ethylene which give, respectively, the preferred reactants, diphenylmethane and 1,2-diphenylethane. Exemplary of other diphenylalkanes are: 1-methyl-1,2-diphenylethane, 1,4-diphenylbutane, 1,6-diphenylhexane, 1,3-diphenylpropane, 2,3-dimethyl-1,4-diphenylbutane, 2-ethyl-3-methyl-1,4-diphenylbutane,2-methyl-1,7-diphenylhexane, 1,9-diphenylnonane and 1,10-diphenyldecane. The diphenylalkane reactant can be produced by various routes. For example, CA 97 38651d (Japanese Kokai 82/45114) and CA 46 7084g disclose the reaction of benzene and ethylene dihalide in the presence of aluminum trichloride to yield diphenylalkane. Another process for producing diphenylalkane includes the oxidative dimerization of toluene at a temperature of at least 400 °C in the presence of a metal oxide catalyst to yield diphenylethane and diphenylalkene. The latter product is then hydrogenated to remove the olefinic unsaturation.

It is not uncommon for the diphenylalkane reactant to be accompanied by various impurities. These impurities often give the final decabromodiphenylalkane product an off color. Exemplary of these color-causing impurities are diphenylmethane, phenylcyclohexane, tetrahydronaphthalene, and the methyl and ethyl derivatives of 1,2-diphenylethane. Diminishing the impurity content can be accomplished in a conventional manner, for example, the diphenylalkane can be recrystallized. See Example VII wherein a recrystallization method is described. Preferably the purified diphenylalkane reactant has less than about 10 wt.% impurities and most preferably, less than about 2 wt.% impurities.

The diphenylalkane which is brominated is, at least initially in a solid form. Thus, the diphenylalkane is at a temperature below its melting point. For essentially pure diphenylethane, the melting point is 53 °C to 55 °C.

The process of this invention is not limited to the use of solid diphenylalkane, as molten diphenylalkane can also be used. When molten diphenylalkane is used, however, an inert atmosphere should be maintained in the reaction vessel containing the molten diphenylalkane in order to reduce the decomposition of the diphenylalkane reactant. Hence, it is easier and more economical to charge the reaction vessel with solid diphenylalkane before feeding the bromine to the vessel.

The amount of elemental bromine ($Br_2$) fed to the reaction vessel should provide sufficient bromine to effect the degree of bromination sought and to provide an easily stirred reaction mass. Generally, from 18 to 30 moles of bromine per mole of diphenylalkane feed will be suitable. Preferably from 20 to 28 moles of

bromine per mole of diphenylalkane are used. A most preferred amount is in the range of from 23 to 27 moles of bromine per mole of diphenylalkane. After the reaction is complete, the bromine not used in the ar-substitution will be a liquid component of the reaction mass and will continue to serve the before-mentioned purpose of providing a stirrable reaction mass.

The bromine addition generally occurs over a period of time and the addition rate is dependent upon the scale of the reaction and the ability to control the temperature and to handle hydrogen bromide evolution. On a laboratory scale, the addition typically requires 0.05 to 1.5 hours while on a commercial scale, the addition could involve 1.0 to 10.0 hours or longer. Four to five hours would be typical for the commercial scale.

It has also been found that the bromine utilized in the process of this invention should contain 10 ppm or less organic impurities, e.g. oil, grease, carbonyl containing hydrocarbons, and iron, so that there is little, if any, impact on the color attributes of the product. Commercial grade bromine having such purity may be available. If such is not available, the organic impurities and water content of the bromine can be conveniently reduced by mixing together a 3 to 1 volume ratio of bromine and concentrated (94-98 percent) sulfuric acid. A two phase mix is formed which is stirred for 10-16 hours. After stirring and settling, the sulfuric acid phase, along with the impurities and water, is separated from the bromine phase. To further enhance the purity of the bromine, the recovered bromine phase can be subjected to distillation.

The bromination catalyst used in the process of this invention is preferably $AlCl_3$ and/or $AlBr_3$, although use may be made of aluminum powder, iron powder, $FeCl_3$, $FeBr_3$, $ZrCl_4$ or any combination of two or more of the foregoing. Other bromination catalysts are suitable, provided that they have sufficient catalytic activity to provide for the extent of bromination called for under the process conditions which will be encountered. Catalytic quantities are used. Typically, the catalysts will be present in an amount within the range of 0.1 to 20 weight percent, based on the weight of the diphenylalkane reactant used in the process. A preferred amount is within the range of from 8 to 15 weight percent on the same basis, with from 9.0 to 11.0 weight percent being most preferred.

The bromination catalyst and diphenylalkane can be charged to the reaction vessel in any order or together. Typically, the bromination catalyst is added to the reaction vessel containing the diphenylalkane to be brominated. However, the bromination catalyst may also be admixed with the bromine and charged to the reaction vessel with the bromine feed.

During the charging of the reaction vessel with the bromine and bromination catalyst, care should be taken not to aspirate atmospheric moisture into the reaction vessel. The presence of moisture in the reaction vessel is detrimental as many bromination catalysts are deactivated by contact with water.

The reaction mass temperature, during the bromine addition is kept below about 15° C, and preferably within the range of from 0° C to 15° C. Since the bromination of diphenylalkane is exothermic, cooling of the reaction mass will be needed to obtain the addition temperature as required above. The heat of reaction can be removed from the reaction mass by cooling the reaction vessel or by having the reaction mass under reflux conditions so that heat can be removed by the use of an overhead condenser. The rate of bromine addition will be dependent upon the ability of the equipment to maintain the selected addition temperature.

After the addition of bromine is at least substantially complete, the reaction mass is brought to a temperature within the range of from 50° C to 60° C. The temperature selected can provide a refluxing condition for the reaction mass, however, a refluxing condition is not necessary. The reaction mass is preferably kept at the selected temperature for that ride time which is needed to obtain an average bromine number of at least about 9.0. The average bromine number is defined as the average number of bromine atoms ar-substituted on each brominated diphenylalkane molecule in the product. Thus, an average bromine number of 9.0 indicates that not all of the diphenylalkane molecules in the product have been ring perbrominated, hence, the presence of the lower bromo homologs, e.g., nonobromodiphenylalkane, and octabromodiphenyl alkane, in the product. As the average bromine number approaches 10.0, the amount of these lower bromo homologs will decrease and the amount of the decabromo homolog will increase.

To obtain the selected average bromine number above 9.0, sampling and analysis of the product, as it is produced, can be used to determine the sufficiency of the ride time. If a substantially perbrominated product is desired, the ride time is conveniently determined by monitoring the HBr evolution from the reaction mass. When the HBr evolution ceases to be detected, no further significant bromination is occurring and thus the ride time has been satisfied. Generally, the ride time will be from 30 minutes to 5 hours, with 2 to 4 hours being preferred.

After the ride time, the reaction mass will comprise a liquid-solid mixture. The solid comprises brominated diphenylalkane, catalyst, entrained bromine and other impurities. The liquid will comprise mostly bromine. The recovery of the brominated diphenylalkane product and its entrained bromine is effected conventionally. For example, the reaction mass can be steam stripped to remove non-entrained bromine

from the reaction mass and to deactivate the catalyst. The remaining solids are then washed with an aqueous base, e.g., an aqueous solution of NaOH or $Na_2CO_3$, to neutralize and remove any HBr present. A final water washing step is used to obtain a product which is predominate, i.e., $50^+$ weight percent, in decabromodiphenyl-alkane. This product is of good color and can be further treated to have superior color. A preferred product is one which contains $90^+$ weight percent, and most preferably $98^+$ weight percent, decabromodiphenylalkane.

The further treatment will generally include the removal of entrained bromine from the product. This removal can be effected by oven-aging the product at a temperature within the range of from 180°C to 300°C for 6 to 20 hours preferably from 200° to 250°C. Another method of treatment comprises contacting the product with an aqueous solution of $Na_2CO_3$ and $Na_2SO_3$ having a molar ratio of about 3:1 $Na_2CO_3$ to $Na_2SO_3$ at a temperature in excess of about 180°C, most preferably in the range of from 200° to 300°C for 30 minutes to 4 hours, most preferably 2 hours.

In another embodiment, solid diphenylalkane can be added to a reaction vessel containing a bromination catalyst and sufficient bromine to effect the degree of bromination sought and to provide an easily stirred reaction mass. As beforementioned, the amount of bromine required is generally in the range of from 18 to 30 moles per mole of diphenylalkane reactant. The rate of diphenylalkane addition to the reaction vessel is dependant on the ability of the equipment to maintain the selected bromination temperature. Generally, the diphenylalkane will be added to the reaction vessel over a period of 5 minutes to 10 hours or more depending on the scale of the equipment utilized to effect the desired bromination reaction. In all other respects, the reaction mass in this embodiment may be treated similarly to the reaction mass obtained by the addition of bromine to solid diphenylalkane in the beforemen-tioned embodiment of the invention.

The decabromodiphenylalkane predominant product of this invention may be used as a flame retardant in formulation with virtually any flammable material. The material may be macromolecular, for example, a cellulosic material or a polymer. Illustrative polymers are: olefin polymers, cross-linked and otherwise, for example, homopolymers of ethylene, propylene, and butylene; copolymers of two or more of such alkylene monomers and copolymers of one or more of such alkylene monomers and any other copolymerizable monomers, for example, ethylene/propylene copolymers, ethylene/ethyl acrylate copolymers and ethylene/vinyl acetate copolymers; polymers of olefinically unsaturated monomers, for example, polystyrene, e.g., high impact polystyrene, and styrene copolymers; polyurethanes; polyamides; polyimides; polycarbonates; polyethers; acrylic resins; polyesters, especially poly(ethyleneterephthalate) and poly-(butyleneterephthalate); epoxy resins; alkyds; phenolics; elastomers, for example, butadiene/styrene copolymers and butadiene/acrylonitrile copolymers; terpolymers of acrylonitrile, butadiene and styrene; natural rubber; butyl rubber; and polysiloxanes. The polymer may also be a blend of various polymers. Further, the polymer maybe, where appropriate, cross-linked by chemical means or by irradiation.

The amount of product used in a formulation will be that quantity needed to obtain the flame retardancy sought. It will be apparent to those skilled in the art that for all cases no single precise value for the proportion of the product in the formulation can be given, since this proportion will vary with the particular flammable material, the presence of other additives and the degree of flame retardancy sought in any given application. Further, the proportion necessary to achieve a given flame retardancy in a particular formulation will depend upon the shape of the article into which the formulation is to be made, for example, electrical insulation, tubing and film will each behave differently. In general, however, the formulation may contain from 5 to 40 wt. percent, preferably 10 to 30 weight percent, of the product when it is the only flame retardant compound in the formulation.

It is especially advantageous to use the product with an inorganic compound, especially ferric oxide, zinc oxide, zinc borate, the oxide of a Group V element, for example, bismuth, arsenic, phosphorus and especially antimony, in the formulation. Of these compounds, antimony oxide is especially preferred. If such a compound is present in the formulation, the quantity of product needed to achieve a given flame-retardancy is accordingly reduced. Generally, the product and the inorganic compound are in a weight ratio of from 1:1 to 7:1, and preferably of from 2:1 to 4:1.

Formulations containing a flame retardant system comprised of the product of this invention and the above inorganic compounds may contain up to about 40 percent by weight of the system and preferably between 20 percent and 30 percent by weight.

Any of the additives usually present in formulations, e.g., plasticizers, antioxidants, fillers, pigments, and UV stabilizers, can be used in formulation with the product of this invention.

Thermoplastic articles formed from formulations containing a thermoplastic polymer and a product of this invention can be produced conventionally, e.g., by injection molding, extrusion molding, or compression molding.

The following Examples merely illustrate the invention described herein and are not to be taken as limiting such inventions.

EXAMPLE I

The example is presented for comparative purposes. The $Br_2$ used was not purified prior to use in the procedure. The diphenylethane reactant was melted and added to the kettle under an ambient atmosphere. The diphenylethane reactant was obtained from Aldrich Chemical Company, Inc. and was recrystallized to enhance its purity.

A 500 Ml resin kettle was equipped with a mechanical stirrer, a reflux condenser, a thermometer with a temperature regulator, an addition funnel wrapped with a heating tape, and a caustic scrubber. The kettle was charged with bromine (400.0 g, 2.5 moles), and cooled to 10°C, using an ice-bath. Anhydrous aluminum chloride (2.1 g) was then added to the kettle. Diphenylethane (18.2 g, 0.1 mole) was melted in the addition funnel to about 80°C. The molten diphenylethane was then added to the kettle in which the contents were at 10-17°C. The addition took about 17 minutes. The kettle contents were heated to reflux (59°C) for 3.5 hours. After refluxing, the kettle contents were cooled to 45°C and water (200 Ml) was added thereto. The kettle contents were allowed to come to room temperature. The kettle contents were then heated and distilled to remove the excess bromine. After bromine removal, the kettle contents were filtered to recover a solid product which was washed with water, and then with 10 percent aqueous Hcl, followed by water again (2 x 200 Ml). The washed product was heated in a forced-air oven at 200°C for 22 hours. The heated product (94.0 g, 96.7 percent yield) had a melting point of 350-356°C and Hunter color values of L = 84.7, a = 0.95, b = 10.12 and Y.I. = 20.9.

EXAMPLE II

This example is presented for comparative purposes. The bromine used was not purified and the diphenylethane was melted and added to the kettle in the presence of air.

A 3-liter resin kettle was equipped with a mechanical stirrer, a thermometer with a therm-o-watch, an addition funnel wrapped with a heating tape, a heating mantel and double reflux condensers. The addition funnel was charged with ground diphenylethane (111.0 g, 0.60 mole), and heated slowly with the heating tape. The reactor was charged with bromine (2407.0g, 15 moles) and catalyst ($AlCl_3$, 8.9 g). Molten diphenylethane (kept at 55-66°C) was then added to the bromine and catalyst over a period of 60-75 minutes. During the addition, the kettle temperature was kept at 25-30°C After the addition was complete, the reaction mixture was stirred and heated at reflux (60°C) for 4.5 hours. Water (1000 Ml)was then charged to the kettle and bromine was distilled off to a vaporhead temperature at 100°C. The product was filtered, washed once with water and then with xylene (250 Ml). The product was oven-aged at 200°C for 16 hours, to give 572.0 g (98 percent) of an off-white solid having Hunter color values of L = 79.59, a = 1.19, b = 11.92 and Yellowness Index (Y.I.) of 28.15. The solid had a melting point of 340-344°C and a bromine content of 82.7 percent.

EXAMPLE III

This example illustrates a process of this invention. The equipment described in Example I was used for this example. The $Br_2$ used was purified by stirring together a mixture of bromine and concentrated $H_2SO_4$ (volume ratio of 3:1) for a period of about 12-14 hours. After the period, a two phase mixture is obtained with purified bromine comprising one of the phases. This bromine phase was recovered. Even though this recovered bromine was very pure, further purification was effected by distilling the bromine.

The kettle was charged with diphenylethane (18.2 g, 0.1 mole), and cooled to 10°C, using an ice-bath. Anhydrous aluminum chloride (1.8 g) was then added to the kettle. Liquid bromine (480.0g, 3.0 moles) at a temperature of 10-16° C was fed into the kettle over a period of 30 minutes. After the bromine addition was complete, the kettle contents were heated to reflux (59°C) for 3 hours. After refluxing, the kettle contents were cooled to 25° C and water (200 mL) was added thereto. The kettle contents were then heated and distilled to a vapor temperature of 100° C to remove the excess bromine. After bromine removal, the kettle contents were filtered to recover a solid product. The solid product was heated in a forced-air oven at 200°C for 24 hours. The heated product (94.0 g, 96.7 percent yield) had Hunter color values of L = 85.0, a = 1.9, b = 10.4 and Y.I. = 23.8.

EXAMPLE IV

The procedure of Example III was followed except that: (1) the kettle contents were refluxed for 30 minutes after the bromine addition was complete. The product was oven-aged at 200° C for 16 hours and then oven-aged at 250° C for 3 hours.

The resultant product (95.7 g, 98.4 percent yield) had Hunter color values of L = 88.0, a = 1.5, b = 8.1 and Y.I. = 18.1.

EXAMPLE V

The procedure of Example III was followed except that after the bromine addition was complete, the reaction mass was stirred at ambient room temperature for 4 hours with no reflux.

The final product (92.4 g, 98.0 percent yield) had Hunter color values of L = 90.6, a = 0.8, b = 10.3 and Y.I. = 21.3.

EXAMPLE VI

The following example illustrates a method for purifying diphenylethane.

A 1-L beaker was charged with methanol (300 mL). Crude diphenylethane (300g) was then added. The contents of the beaker were heated and stirred at 65°C, and the resulting clear solution was then allowed to cool slowly to room temperature. A crystalline solid was formed. The solid was filtered and washed once with 120 mL methanol and then dried. The recovery was 274.5 g (91.5%). The recrystallized material had a melting point of 50°C- 54°C which is slightly higher than the 49°C-50°C for the original starting diphenylethane. The starting diphenylethane had a Y.I. of 33.2 (L = 81.2, a = -2.9, b = 16.1) while the recrystallized diphenylethane material had a Y.I. of 2.8. (L = 90.8, a = -0.4, b = 1.4).

## Claims

1. A process for preparing a product predominate in decabromodiphenylalkane and having an average bromine number of at least about 9.0, the process comprising: forming a stirrable reaction mass by feeding bromine to a reaction vessel containing diphenylalkane, which diphenylalkane is, at least initially, in solid form, and a bromination catalyst, the bromine (i) containing about 10 ppm or less impurities, and (ii) being charged in an amount which provides from 18 to 30 moles of bromine per mole of diphenylalkane fed; maintaining the reaction mass at a temperature which is less than or equal to about 15°C during the feeding; subsequent to the feeding, obtaining a reaction mass temperature within the range of from 50°C to 60°C; and recovering from the reaction mass the decabromodiphenylalkane predominant product.

2. The process of Claim 1 wherein the product comprises about $90^{+}$ weight percent decabromodiphenylalkane.

3. The process of Claim 1 or 2 wherein the diphenylalkane reactant is diphenylethane.

4. The process of any of Claims 1-3 wherein the diphenylalkane reactant contains less than about 2 wt.% impurities, the amount of bromine charged provides from 20 to 28 moles of bromine per mole of diphenylalkane, and the reaction mass temperature which is obtained subsequent to the bromine feed is maintained from 5 minutes to 5 hours.

5. The process of any of the preceding claims wherein the product is recovered by steam stripping the reaction mass to remove non-entrained bromine therefrom, the recovered product is washed with an aqueous base to neutralize any BHr which may be present, the washed product is treated with a chelating agent to facilitate the removal of any deactivated catalyst therefrom and the treated product is washed with an aqueous medium.

6. The process of Claim 5 wherein the aqueous medium washed product contains $95^{+}$ weight percent decabromodiphenylalkane and is further treated to remove entrained bromine therefrom.

7. The process of Claim 5 wherein the aqueous medium washed product is heated to a temperature above about 70°C to release entrained bromine therefrom.

**8.** The process of Claim 5 wherein the aqueous medium washed product is contacted with an aqueous treatment solution of $Na_2CO_3$ and $Na_2SO_3$ having a molar ratio of $Na_2CO_3$ to $Na_2SO_3$ of about 3:1 at a temperature of about 180 ° C or more.

**9.** A process for preparing a product predominate in decabromodiphenylalkane and having an average bromine number of at least about 9.0, the process comprising: forming a stirrable reaction mass by feeding solid diphenylalkane to a reaction vessel containing bromine and a bromination catalyst; maintaining the reaction mass at a temperature which is less than or equal to about 15 ° C during the feeding; subsequent to the feeding, obtaining a reaction mass temperature within the range of from 50 ° C to 60 ° C; and recovering from the reaction mass the decabromodiphenylalkane predominant product.

**10.** The process of Claim 9 wherein the diphenylalkane reactant is diphenylethane, the amount of bromine in the reaction vessel provides from 20 to 28 moles of bromine per mole of diphenylalkane, and the decabromodiphenylalkane predominant product is oven-aged at a temperature within the range of from 200 ° C to 250 ° C for 6 to 20 hours.

European Patent Office

# EUROPEAN SEARCH REPORT

Application Number

EP 91120774.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP - A - 0 347 116 (ETHYL CORPORATION) * Examples I,III; claims * -- | 1-3,7, 9,10 | C 07 C 25/18<br>C 07 C 17/12 |
| A | GB - A - 2 097 383 (GREAT LAKES CHEMICALS) * Example I; claims * -- | 9 | |
| A | PATENT ABSTRACTS OF JAPAN, unexamined applications, C section, vol. 3, no. 72, June 21, 1979 THE PATENT OFFICE JAPANESE GOVERNMENT page 22 C 49 * Kokai-no. 54-44 623 (ASAHI GLASS) * -- | 1-3 | |
| P,A | EP - A - 0 447 152 (ETHYL CORPORATION) * Example III; claims * ---- | 1-3,5-7,9,10 | TECHNICAL FIELDS SEARCHED (Int. Cl.5)<br>C 07 C 25/00<br>C 07 C 17/00<br>C 07 C 43/00 |

The present search report has been drawn up for all claims

| Place of search | Date of completion of the search | Examiner |
|---|---|---|
| VIENNA | 03-02-1992 | KÖRBER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document